Europäisches Patentamt

European Patent Office

Office européen des brevets

(19)

(11) Veröffentlichungsnummer : **0 373 178 B1**

(12) **EUROPÄISCHE PATENTSCHRIFT**

(45) Veröffentlichungstag der Patentschrift :
**19.08.92 Patentblatt 92/34**

(51) Int. Cl.⁵ : **A61F 2/04,** A61M 25/00,
A61M 25/01, F16K 1/20

(21) Anmeldenummer : **89900049.1**

(22) Anmeldetag : **07.12.88**

(86) Internationale Anmeldenummer :
**PCT/EP88/01113**

(87) Internationale Veröffentlichungsnummer :
**WO 89/05127 15.06.89 Gazette 89/13**

(54) **ANTIREFLUXIVE HARNLEITERSCHIENE MIT BLASENSEITIGEM, DYNAMISCHEM HAUBENVENTIL.**

(30) Priorität : **10.12.87 DE 3741832**

(43) Veröffentlichungstag der Anmeldung :
**20.06.90 Patentblatt 90/25**

(45) Bekanntmachung des Hinweises auf die
Patenterteilung :
**19.08.92 Patentblatt 92/34**

(84) Benannte Vertragsstaaten :
**AT BE CH DE FR GB IT LI LU NL SE**

(56) Entgegenhaltungen :
**EP-A- 0 208 841**
**WO-A-80/01507**
**DE-A- 3 339 179**
**DE-B- 1 250 704**
**DE-U-86 140 132**

(56) Entgegenhaltungen :
**FR-A- 2 133 083**
**FR-A- 2 409 747**
**US-A- 3 999 551**
**US-A- 4 334 327**
**US-A- 4 512 770**
**US-A- 4 574 806**
**US-A- 4 685 905**

(73) Patentinhaber : **HOENE, Eberhard**
**Warburghof 16**
**W-3000 Hannover 61 (DE)**

(72) Erfinder : **HOENE, Eberhard**
**Warburghof 16**
**W-3000 Hannover 61 (DE)**

(74) Vertreter : **Leine, Sigurd, Dipl.-Ing. et al**
**LEINE & KÖNIG Patentanwälte**
**Burckhardtstrasse 1**
**W-3000 Hannover 1 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 373 178 B1

**Beschreibung**

Die Erfindung betrifft eine antirefluxive, innere Harnleiterschiene gemäß Oberbegriff des Anspruchs 1. Innere Harnleiterschienen werden üblicherweise zur Ableitung von Urin aus dem Nierenbecken-Kelchsystem in die Blase verwendet, wenn der normale Harntansport -

a) durch Einengung des Harnleiters, z. B. durch Narbenbildung, Verdrängung von außen oder angeborene Engstellung, oder

b) durch Verlegung der Harnleiterlichtung, z. B. durch Harnstein, Gerinnsel oder Tumor -

beeinträchtigt ist. Damit soll drohendem Schaden für das Funktionsgewebe der Niere durch anhaltende Druckbelastung bzw. lebensgefährlicher Infektion der im oberen Hohlsystem "stehenden Gewässer" vorgebeugt werden.

Bisher eingesetzte innere Harnleiterschienen sind schlauchförmige Kunststoffkatheter, die an ihren Enden, oben mit einem kleineren Radius als unten, ringelförmig gekrümmt sind, um durch Einhaken vor dem Harnleiterabgang bzw. nach der Einmündung in die Blase ein Verrutschen nach unten oder oben zu verhindern. Die Schienen weisen entlang ihres Verlaufs, und vor allem an den Abschnitten, die im Nierenbecken bzw. in der Blasenlichtung zu liegen kommen, mehrere seitliche (Stanz-)Löcher auf, um den Zu- und Abfluß auch bei längerer Verweildauer und Verkrustung einzelner Öffnungen zu gewährleisten. Zur Lagekontrolle sind die Kunststoffkatheter im Röntgen kontrastgebend, wobei der gesamte Schlauch oder ein Strang im Verlauf aus strahlendichtem Material besteht.

Die Einlage gebräuchlicher innerer Harnleiterschienen erfolgt durch Einschub eines erheblich längeren und deutlich steiferen Führungsdrahtes, wodurch die vorerwähnten Krümmungen begradigt werden. Rückläufig werden die Schienenkatheter unter Sicht- und Durchleuchtungskontrolle über den Arbeitseinsatz eines Blasenspiegels in die untere Harnleiterausmündung - das Ostium - eingefädelt und mit dem Führungsdraht den Harnleiter hinaufgeschoben. Der Führungsdraht zieht durch einen weiteren Kunststoffschlauch, der vom Ende des Harnleiterkatheters bis kurz vor das Ende des Führungsdrahtes reicht, dessen Ende somit freigelassen bleibt. Bei regelrechter Lage des inneren Harnleiterkatheters wird das freiliegende Ende des Kunststoffschlauches mit der Hand fixiert und der Führungsdraht durch die Lichtung herausgezogen. Dabei stützt sich das blasenseitige Schienenende am Kunststoffschlauch ab, damit die Reibung in den Krümmungsbereichen den Schienenkatheter nicht mit dem Führungsdraht aus dem Harnleiter herauszieht. Dies geschieht unter Sichtkontrolle mit dem Blasenspiegel; sobald der Führungsdraht nur noch im äußeren Kunststoffschlauch liegt, ist das blasenseitige Schienenende freigegeben und ringelt sich der Krümmung gemäß in der Blasenlichtung auf.

Die Sicherung des Harnabflusses durch gebräuchliche innere Katheterschienen wird aber bisher mit zeitweilig wiederkehrenden, unphysiologischen Druckspitzen im oberen Hohlsystem erkauft. Die freie Durchgängigkeit dieser Schienen hebt die körpereigene Ventilfunktion der unteren Harnleiterausmündung in die Blase, die vor Harnrückfluß schützt, auf. Damit wird eine Drucksteigerung in der Blasenlichtung durch zurückfließenden Urin (Reflux) kontinuierlich auf das Nierenbecken-Kelchsystem übertragen. Dies führt bei Trägern solcher Schienen oft zu erheblichen Schmerzen. Einerseits aktiviert die plötzliche, fortgeleitete Drucksteigerung beim Wasserlassen Dehnungsrezeptoren in der Nierenbeckenwand, deren Reizung, wie bei Koliken, als Schmerz empfunden wird, zum anderen führen Fremdkörper in der Blase, und das blasenseitige Ende solcher Schienenkatheter ist ein Fremdkörper, häufig zu schmerzhaften Blasenkrämpfen durch Wandreizung; die fortgeleiteten Druckwellen werden auf der durch Katheterschiene entlasteten Seite als Flankenschmerzen wahrgenommen. Zum Ausgleich obstruktiver Blasenentlerungsstörungen sind diese Patienten oft auch mit Blasendauerkatheter versorgt. Diese stellen eine Eintrittspforte für Keime dar, welche dann mit dem infizierten Urin den inneren Harnleiterkatheter entlang ungehindert aufsteigen können und gelegentlich lebensbedrohliche obere Harnwegsentzündungen mit Beteiligung des Nierengewebes hervorrufen.

Antirefluxventile, die im Ruhezustand geschlossen sind, setzen zum Beispiel durch ihre Steifheit dem Harnabfluß einen übermäßigen Strömungswiderstand entgegen; sind sie weicher, so besteht die Gefahr, daß sie an der Blasenwand abknicken und sich daraufhin überhaupt nicht mehr öffnen können. Ventilformen, die in der Katheterlichtung angebracht werden, setzen ebenfalls den Strömungswiderstand herauf oder haben nach kurzer Zeit durch Verkrustung infolge von Turbulenzen hinter dem Ventil ihre Schließfähigkeit bzw. Offnungsfreiheit, eingebüßt.

Aus der EP-A-0 208 841 ist ein Harnleiterkatheter bekannt, der am blasenseitigen Ende des Katheterschlauches ein Rückschlagventil in Form eines Folienventils aufweist. Das Folienventil besteht aus zwei flach aufeinanderliegenden dünnwandigen Folienzuschnitten, die an ihren seitlichen Enden miteinander verklebt oder verschweißt sind, so daß das Folienventil die Öffnung des Harnleiterkatheters im Ruhezustand verschließt. Dieses Ventil weist einen relativ hohen Öffnungsdruck auf, so daß die Gefahr eines gesundheitsschädlichen Harnrückstaues besteht.

Die Aufgabe der vorliegenden Erfindung besteht darin, eine innere Harnleiterschiene der eingangs ge-

EP 0 373 178 B1

nannten Art so auszubilden, daß die blasenseitigen Öffnungen der Katheterschiene durch ein einziges, endständiges, dynamisches Sperrventil gegen Rückfluß ersetzt werden. Die Ausbildung soll dergestalt sein, daß die verschiedenen Nachteile bisheriger Konzepte auf die vorliegende Erfindung nicht zutreffen.

Diese Aufgabe wird durch die Ausbildung gemäß Kennzeichen des Anspruchs 1 gelöst.

Vorteilhafte und zweckmäßige Weiterbildungen der erfindungsgemäßen Aufgabenlösung sind in den Unteransprüchen gekennzeichnet.

Durch die Weiterbildung nach Anspruch 11 wird die antirefluxive, innere Harnleiterschiene mit einem Zusatz versehen, der das zarte Haubenventil bei der antegraden Einlage von oben, z. B. bei offenen Schnittoperationen, gegen Beschädigung schützt.

Das hier vorgestellte Ventil zeichnet sich im Unterschied zu früheren Ventilen, die deshalb nicht zur klinischen Anwendung gekommen sind, dadurch aus, daß es

a) im Ruhezustand als Fortsetzung des vollständigen Katheterquerschnittes geöffnet vorliegt;
b) infolge eines hydrodynamischen Wirkprinzips, beim Ansatz von Harnrückfluß, annähernd trägheitsfrei geschlossen wird, wenn der Blaseninnendruck den im Harnleiter und im oberen Hohlsystem übersteigt;
c) nicht durch Verformung oder Abknickung von außen verstopft werden kann;
d) durch erhebliche Verformung beim Schließen, wie beim Öffnen, von Ablagerungskrusten befreit wird, wodurch die Funktionsfähigkeit auch bei längerer Verweildauer gewährleistet bleibt.

Die Erfindung soll nachfolgend anhand der beigefügten Zeichnungen näher erläutert werden.

Es zeigt:

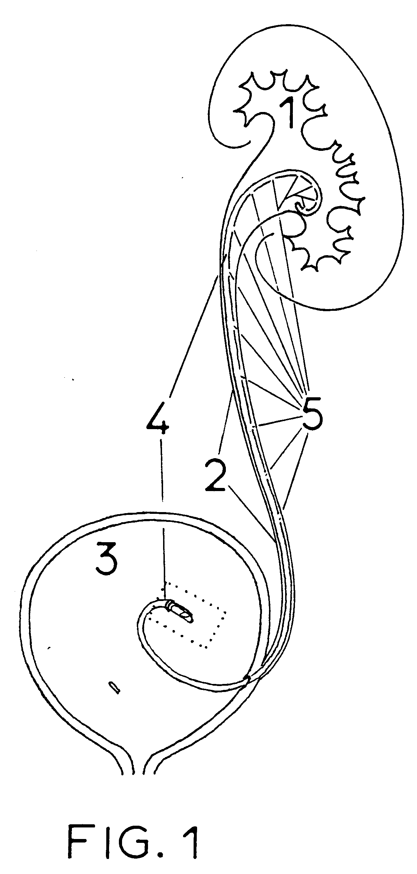

Abb. 1 in der Draufsicht von vorn schematisch die regelrechte Lage eines antirefluxiven inneren Harnleiterkatheters vom Nierenbecken-Kelchsystem bis in die Blase;

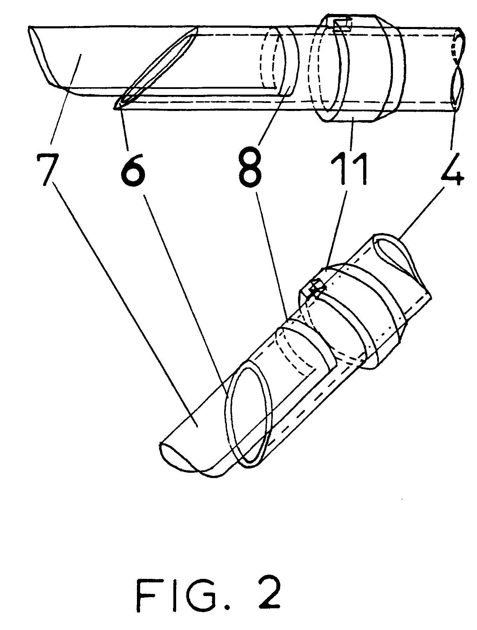

Abb. 2 als Ausschnittvergrößerung das blasenseitige Ende der Katheterschiene mit dem antirefluxiven Haubenventil;

Abb. 3 als weitere Ausschnittvergrößerung die Verformung des besagten Ventils beim Ansatz von Rückfluß, sowie im geschlossenen Zustand;

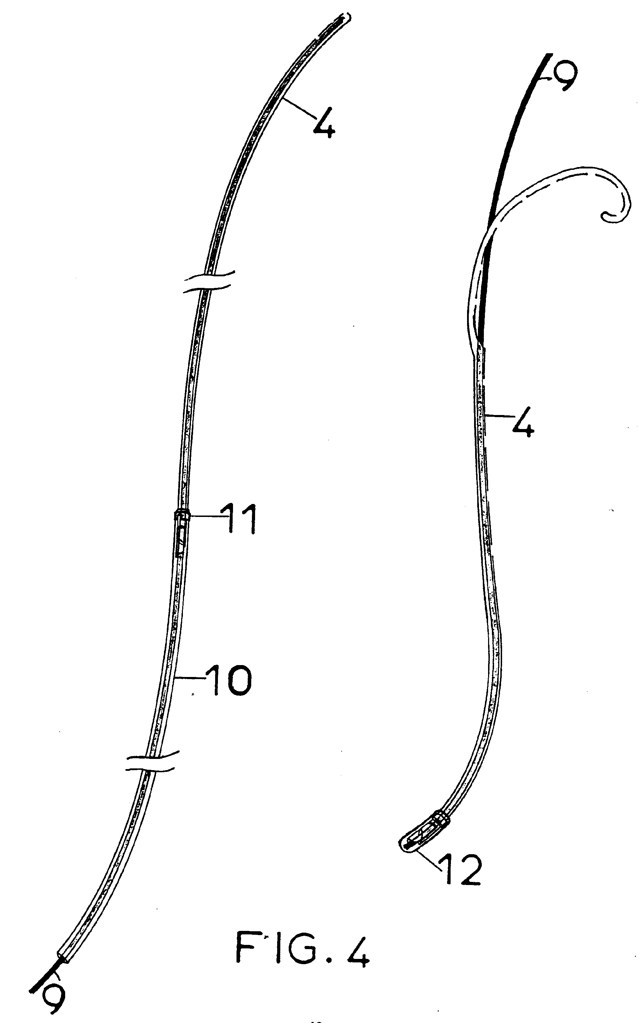

Abb. 4 als schematische Darstellung die Einlage der antirefluxiven inneren Harnleiterschiene unter Verwendung eines Führungsdrahtes und

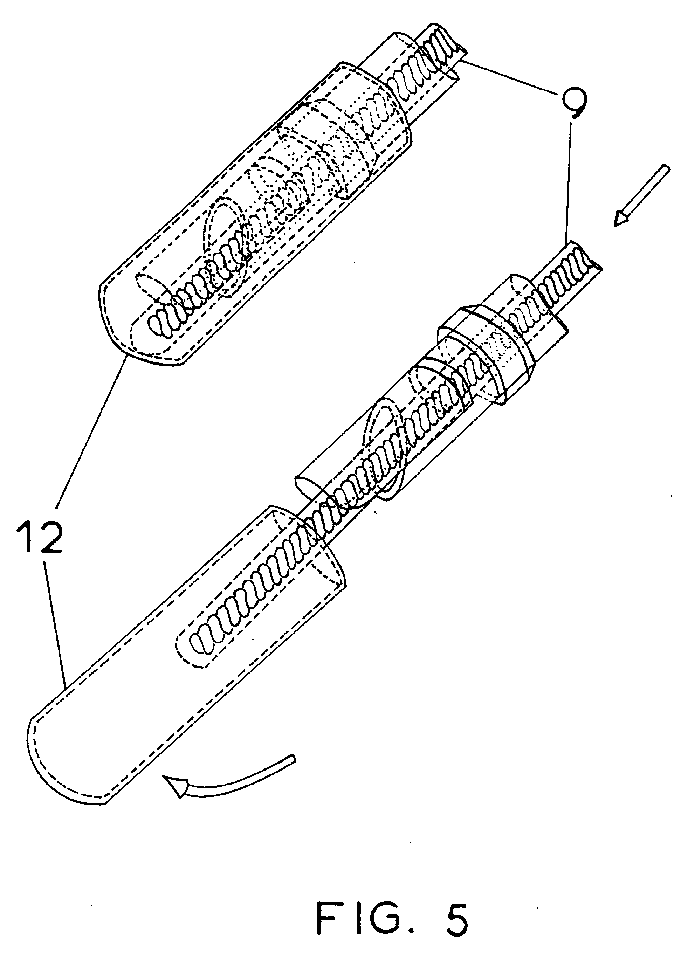

Abb. 5 als Ausschnittvergrößerung die Schutzkappe für antegrade Einlage, sowie deren Entfernung bei regelrechter Lage des antirefluxiven inneren Harnleiterkatheters.

Erfindungsgemäß weist der antirefluxive innere Harnleiterkatheter 4 nur in den oberen und mittleren Abschnitten, d. h. im Nierenbecken-Kelchsystem 1 und im Verlauf des Harnleiters 2, seitliche Perforationen 5 auf. Der untere Abschnitt, der in der Blase 3 zu liegen kommt, hat keine seitlichen Perforationen, nur das mit einer in die Katheterkrümmung weisenden Schrägung auslaufende, blasenseitige Ende 6 ist im Ruhezustand des Ventils in voller Lichtungsweite offen. Im ausreichenden Abstand, um die zum Schließen erforderliche Verformung zu gewährleisten, ist die Folie des Haubenventils 7 am Katheterumfang befestigt 8.

Das Haubenventil 7 besteht aus Kunststoffolie von annähernd rechteckiger Fläche. Die Breite beträgt etwa zwei, die Länge etwa drei Außendurchmesser des mit Ventil zu bestückenden Harnleiterkatheters. Am einen Breitenrand ist ein schmaler (1/4 - 1/3 Katheterdurchmesser) Streifen 8 zur Befestigung der Folie am inneren Harnleiterkatheter vorgesehen, die gegenüberliegenden Ecken sind abgerundet. Durch die Befestigung auf dem der Schrägung zugewandten Umfangsteil des Katheters, etwa einen Außendurchmesser des Katheters vor dem Ende, liegt die Kunststoffolie dem Umfang des Katheters außen an und besitzt im Ruhezustand die beschriebene Ausbildung als Haubenventil 7, welches die blasenseitige Katheterausmündung um etwa einen Katheterdurchmesser überragt.

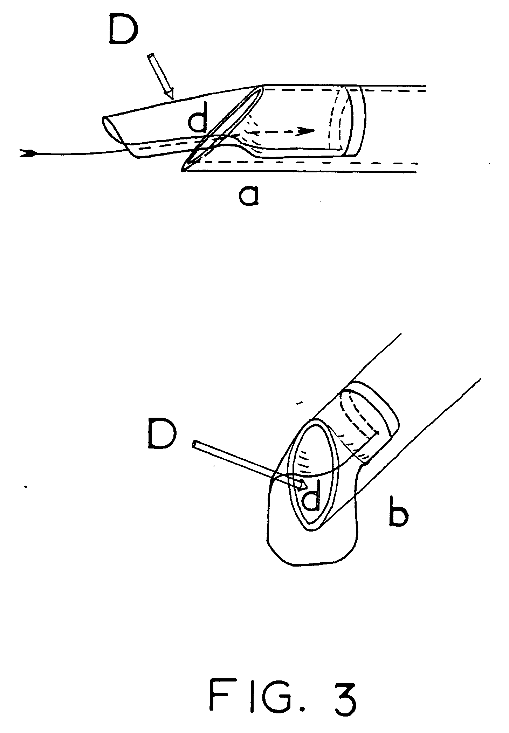

Der Ventilverschluß erfolgt durch das nach VENTURI benannte hydrodynamische Prinzip (Abb. 3a, b). Bei ansteigendem Blasendruck führt schon der Ansatz von Harnrückfluß zur Durchströmung des ventilbestückten, blasenseitigen Katheterendes, mit einer Druckminderung hier im Vergleich zum restlichen Blaseninnendruck. Der Druckgradient zwischen dem ambienten Blaseninnendruck (D) und dem innerhalb des durchströmten Ventils (d) nimmt die leichte Folienhaube unter Abflachung und seitlicher Verformung mit und legt sie auf die endständige Katheteröffnung, wo sie diese verschließt, bis der äußere Überdruck, z. B. am Ende der Blasenentleerung, wieder nachläßt. Dann führt die Elastizität der Folienhaube sie wieder in die Ausgangskrümmung zurück und gibt somit das Schienenende frei. Die wiederkehrende Verformung beim Schließen und Öffnen sprengt entstehende Krusten frühzeitig von der Folie ab.

Die Folienstärke muß ausreichen, dem Druckunterschied im geschlossenen Zustand zu widerstehen und um eine Einstülpung der Folie in die verschlossene Katheteröffnung zu verhindern. Die ihr dann innewohnende Rigidität gewährleistet die Rückkehr in die Krümmung des Katheterumfangs, an der das Ventil befestigt ist, wodurch das Haubenventil im Ruhezustand voll geöffnet bleibt. Andererseits muß die Folie ausreichend leicht und flexibel sein, schon einem geringen Druckgefälle zu folgen, sich durch Abflachung zu verformen, und die schräge terminale Katheteröffnung abzudecken.

Die Folienhaube umfaßt den Katheter nicht im vollen Umfang, sondern läßt den der Schrägung abgewandten Umfangsteil - etwa ein Drittel des Gesamtumfangs - frei. Diese Tatsache hat zwei Gründe. Eine Teilummantelung dieser Ausbildung reicht auch bei seitlicher Verschiebung aus, die Katheteröffnung sicher abzudeckeln, und ist zudem leichter, unter Abflachung zu verformen. Darüber hinaus schützt der breite, offene Spalt gegen unerwünschte Abflußstörung, z. B. durch Abknicken oder Verkanten des Haubenventils an der Blasenwand.

Zum Einführen des antirefluxiven inneren Harnleiterkatheters mittels Blasenspiegel, durch die Harnröhre von unten in den Harnleiter bis ins obere Hohlsystem hinauf, wird die Katheterschiene durch einen in ihre Lichtung eingeführten Führungsdraht 9 begradigt, der sich an ihrem stumpf verschlossenen oberen Ende abstützt. Bei regelrechter Lage der inneren Harnleiterschiene wird der Führungsdraht herausgezogen. Damit die Schiene nicht mitgeht, kommt bei herkömmlichen Systemen ein weiterer Kunststoffschlauch zum Einsatz, der vom blasenseitigen Schienenende nicht ganz bis zum Ende des Führungsdrahtes reicht. Dieser zweite Schlauch, im folgenden als Sperrschlauch 10 bezeichnet, wird außen mit der Hand fixiert, während der Führungsdraht durch seine Lichtung herausgezogen wird, und verhindert das Mitlaufen der Harnleiterschiene, die bei herkömmlichen Modellen endständig abgestützt wird. Das ist hier nicht möglich, weil es zur Beschädigung des zarten Haubenventils kommen könnte.

Der Sperrschlauch 10 wird erfindungsgemäß mit einem Innendurchmesser gewählt, der den Außendurchmesser der Schiene mit Haubenventil geringfügig übersteigt, also das blasenseitige Ende der Schiene aufnehmen kann. An der Schiene selbst dient eine ringförmige Sperre 11 knapp oberhalb des Haubenventils, oder in Höhe des Befestigungsrandes 8, als Anschlagring für den Sperrschlauch, während der Führungsdraht herausgezogen wird. Sobald dieser nur noch im Sperrschlauch liegt, kann er unter Sichtkontrolle mit dem Zystoskop dazu verwendet werden, das blasenseitige Schienenende aus dem Sperrschlauch herauszubugsieren.

Der Anschlagring 11 ist mit einer randständigen, rechteckigen Aussparung versehen, die einen korrespondierenden Vorsprung am Rand des Sperrschlauchs 10 aufnimmt, wodurch bei der Einlage der Harnleiterschiene 4 eine Lenkbarkeit erreicht wird. Eine Drehung am freiliegenden Ende des Sperrschlauchs 10 führt dann zu einer entsprechenden Drehung der Harnleiterschiene 4 mit dem Führungsdraht 9 und läßt somit die Richtung des nierenseitigen Schienenendes bestimmen.

Bei Einlage der Harnleiterschiene von oben wird erfindungsgemäß das Haubenventil durch eine wasserlösliche Gelkappe 12 geschützt. Diese umfaßt locker das Schienenende mit dem Ventil und liegt dem Anschlagring reibschlüssig an. Bei regelrechter Lage der Schiene wird die Kappe mit dem Führungsdraht in der Blase vom Anschlagring weggestoßen, gibt das Ventil frei und löst sich bald auf.

## Patentansprüche

1. Antirefluxive innere Harnleiterschiene als nierenseitig geschlossener, mit seitlichen Perforationen (5) nur im oberen und mittleren Abschnitt versehener Kunststoffkatheter, dessen blasenseitige Öffnung mit einem Ventil (7), zur Verhinderung von Harnrückfluß versehen ist, **dadurch gekennzeichnet**, daß das Ventil (7) ein Ruhezustand offenes, beim Ansatz von Rückfluß die blasenseitige Öffnung des Kunststoffkatheters verschließendes Haubenventil mit einem flexiblen, teilzylindrischen Ventilkörper ist.

2. Harnleiterschiene nach Anspruch 1, **dadurch gekennzeichnet**, daß das Haubenventil (7) eine im Abstand von der unteren Katheterausmündung randständig fixierte (8), auf dem äußeren Katheterumfang anliegende Kunststoffolie als Ventilkörper aufweist, die das Schlauchende überragt und sich durch Unterdruck bei rückläufigem Durchfluß an die blasenseitige Katheterausmündung anlegt.

3. Harnleiterschiene nach Anspruch 2, dadurch gekennzeichnet, daß die blasenseitige Katheterausmündung durch ein schräg geschnittenes Schlauchende gebildet wird.

4. Harnleiterschiene nach Anspruch 3, dadurch gekennzeichnet, daß die Schrägung des Schlauchendes in die Krümmung des blasenseitigen Katheterabschnittes zeigt.

5. Harnleiterschiene nach Anspruch 4, dadurch gekennzeichnet, daß daß das Haubenventil (7) auf dem der Schrägung zugewandten Umfangsteil des Katheters angeordnet ist.

6. Harnleiterschiene nach einem der Ansprüche 2 bis 5, dadurch gekennzeichnet, daß die Kunststoffolie des Haubenventils (7) den Schlauchumfang nicht ganz umschließt, sondern den der Schrägung am blasenseitigen Ende abgewandten Umfangsteil partiell freiläßt.

7. Harnleiterschiene nach Anspruch 6, dadurch gekennzeichnet, daß die Kunststoffolie den Schlauchumfang zu etwa zwei Dritteln umfaßt.

8. Harnleiterschiene nach einem der vorhergehenden Ansprüche, dadurch gekennzeichnet, daß für einen an sich bekannten Sperrschlauch (10) zur Entfernung eines üblicherweise verwendeten Führungsdrahtes (9) nach erfolgter Einlage der Harnleiterschiene (4) von unten ein Widerlager vorgesehen ist, das als passender Anschlagring (11) ausgebildet und knapp oberhalb oder in Höhe des Befestigungsrandes (8) vom Haubenventil (7) angeordnet ist.

9. Harnleiterschiene nach Anspruch 8, dadurch gekennzeichnet, daß der Anschlagring (11) eine randständige, rechteckige Aussparung aufweist, die einen korrespondierenden, rechteckigen Vorsprung am Rand des Sperrschlauches (10) aufnimmt und eine kongruente Drehung vom Harnleiterkatheter (4) mit dem Sperrschlauch (10) gewährleistet.

10. Harnleiterschiene nach Anspruch 7 oder 8, dadurch gekennzeichnet, daß der Innendurchmesser des Sperrschlauches (10) geringfügig größer ist als der Außendruchmesser der Harnleiterschiene mit Ventil.

11. Harnleiterschiene nach einem der vorhergehenden Ansprüche, dadurch gekennzeichnet, daß zum Schutz des Haubenventils (7) beim antegraden Einführen eine reibschlüssig blasenseitig übergestülpte, wasserlösliche Gelkappe (12) vorgesehen ist, die nach Eintritt in die Blase mit dem Führungsdraht (9) wegstoßbar ist, um das Haubenventil freizugeben, bevor der Führungsdraht (9) zurückgezogen wird.

## Claims

1. Antirefluxive internal ureteral stent as a catheter of synthetic material, closed at its renal end, with lateral perforations (5) only in the upper and middle sections thereof, and whose vesical end is equipped with a valve for prevention of urinary reflux, wherein said valve (7) is a hood valve with a flexible valve member generally C-shaped in cross section and open in its resting state, said valve member closing the vesical end of the synthetic catheter upon inception of reflux.

2. The ureteral stent of CLAIM 1, wherein said hood valve (7) comprises a synthetic, membranous valve member, affixed to the said lower catheter end opening at some distance (8) from the terminus, extending past the end of the catheter and closing upon the same by means of the pressure differential resulting from retrograde influx to the catheter.

3. The ureteral stent of CLAIM 2, wherein said vesical catheter end is inclined.

4. The ureteral stent of CLAIM 3, wherein said inclination of said catheter end faces into the curvature of the vesical portion of the catheter.

5. The ureteral stent of CLAIM 4, wherein said hood valve member (7) is affixed to the portion of the catheter circumference opposite said inclination.

6. The ureteral stent of CLAIMS 2 to 5, wherein the synthetic membrane of the hood valve does not encompass the entire circumference of said catheter, but rather leaves the portion opposite the inclination at the vesical end partially free.

7. The ureteral stent of CLAIM 6, wherein said valve member encompasses approximately two thirds of said catheter circumference.

8. The ureteral stent (4) of prior claims, wherein a stop means is provided for in the form of an abuttment ring (11) for the tubular stop member (10) - knewn as such - used during customary extraction of the guidewire (9) after correct positioning of the stent, said stop means being affixed to the catheter circumference slightly above or at the level of the hood valves (7) edge of fixation (8).

9. The ureteral stent of CLAIM 8, wherein the abuttment ring (11) inccludes a rectangular notch to accommodate a corresponding rectangular projection at the edge of said stop means (10), providing for congruent rotation of said stent (4) with said tubular stop means (10).

10. The ureteral stent of CLAIMS 7 or 8, wherein the inner diameter of the tubular stop means (10) slightly exceeds the outer diameter of the stent with said valve.

11. The ureteral stent of the prior claims, wherein a water-soluble gelatine cap ((12) is provided for, holding by friction to cover and protect the hood valve (7), and which may be pushed away by means of the guidewire (9), after emplacement in the bladder, to free the hood valve prior is removal of the guidewire.

## Revendications

1. Sonde urétérale interne anti-refluxive sous forme d'un cathéter à bout rénal fermée avec les perforations latérales (5) sur la partie supérieure et au milieu seulement, dont l'ouverture vésicale porte une valve (7) pour éviter le reflux d'urine, **caractérisée en ce que** la valve (7) se compose d'un corps de valve sémi-cylindique flexible et d'une valve à calotte qui est ouverte en état de repos et qui ferme l'ouverture vésicale du cathéter plastique lors d'un reflux.

2. Sonde urétérale suivant la revendication 1, **caractérisée en ce que** la valve à calotte (7) se compose d'un corps de valve sous forme d'urne feuille plastiquue (8) ajustée sur la périphérie du cathéter et fixée à distance de l'ouverture terminale du cathéter sur la paroi de celui-ci, qui surpasse le bout du tuyau et s'accoste à l'ouverture vésicale du cathéter par pression négative lors d'un reflux.

3. Sonde urétérale suivant la revendication 2, **caractériséeen ce que** l'ouverture vésicale du cathéter est formée par une terminaison de tuyau coupée en biais.

4. Sonde urétérale suivant la revendication 3, **caractérisée en ce que** l'inclinaison de la termianison du cathéter est orientée vers la courbure de la partie vésicale du cathéter.

5. Sonde urétérale suivant la revendication 4, **caractérisée en ce que** la valve à calotte (7) est disposée sur la partie périmétrale du cathéter orientée vers l'inclinaison terminale.

6. Sonde urétérale suivant les revendications 2 à 5, **caractérisée en ce que** la feuille plastique de la valve à calotte (7) ne cerne pas tout le périmètre du tuyau, mais laisse ouvert partiellement la partie périmétrale orientée en opposition de l'inclinaison terminale au bout vésical.

7. Sonde urétérale suivant la revendication 6, **caractérisée en ce que** la feuille plastique cerne le périmètre du tuyau à deux tiers environ.

8. Sonde urétérale suivant une des revendications précédentes, **caractérisée en ce qu'**il a été prévu une culée - sous forme d'un anneau d'appui (11) à gaine au dessus ou à la hauteur du bord de fixation (8) de la valve à calotte (7)pour un tuyau de protection (10) connu, utilisé, après mise en place de la sonde urétérale, pour l'extraction de la sonde-guide métallique (9) habituellement utilisée.

9. Sonde urétérale suivant la revendication 8, **caractérisée en ce que** l'anneau d'appui (11) montre une fenestration latérale rectangulaire qui reçoit un ressort rectangulaire correspondant sur la paroi du tuyau de protection (10) et assure un tour congruent du cathéter urétéral (4) avec le tuyau de protection (10).

10. Sonde urétérale suivant les revendications 7 ou 8, **caractérisée en ce que** le diamètre intérieur du tuyau de protection (10) est légèrement supérieur au diamètre extérieur de la sonde vésicale avec valve.

11. Sonde urétérale suivant les revendications précédentes, **caractérisée en ce qu'**il a été prévu pour protéger la valve à calotte lors de l'introduction antérograde, un bonnet (12) en gel hydrosoluble qui peut être repoussé à l'aide de la sonde-guide métallique (9) après l'entrée dans la vessie, pour libérer la valve à calotte avant de retirer la sonde-guide (9).

FIG. 1

FIG. 2

FIG. 3

4

9

11

4

10

9

12

FIG. 4

FIG. 5